# EUROPEAN PATENT SPECIFICATION

(11) **EP 4 338 777 B1**
(45) Date of publication and mention of the grant of the patent: **12.08.2026**
(21) Application number: 22806141.2
(22) Date of filing: 13.05.2022
(51) Int. Cl.: A61M 16/10, A62B 23/02, A01N 59/16, A61M 16/08

(54) **BIOCIDAL HEAT AND MOISTURE EXCHANGE FILTER, METHOD FOR PREPARING A BIOCIDAL HEAT AND MOISTURE EXCHANGE FILTER, AND USE OF SILVER NANOPARTICLES**
BIOZIDER WÄRME- UND FEUCHTIGKEITSAUSTAUSCHFILTER, VERFAHREN ZUR HERSTELLUNG EINES BIOZIDEN WÄRME- UND FEUCHTIGKEITSAUSTAUSCHFILTERS UND VERWENDUNG VON SILBERNANOPARTIKELN
FILTRE ÉCHANGEUR DE CHALEUR ET D'HUMIDITÉ À ACTIVITÉ BIOCIDE, PROCÉDÉ DE PRÉPARATION DE FILTRE ÉCHANGEUR DE CHALEUR ET D'HUMIDITÉ À ACTIVITÉ BIOCIDE ET UTILISATION DE NANOPARTICULES D'ARGENT

(30) Priority: 13.05.2021 BR 102021009290
(43) Date of publication of application: 20.03.2024
(73) Proprietor: MLA Suprimentos Medicos Ltda, 13213009 Jundiai - Sp (BR)
(72) Inventor: THIPE, Velaphi Clement, 13213-009 JUNDIAI - SP (BR); BATISTA, Jorge Gabriel dos Santos, 13213-009 JUNDIAI - SP (BR); LUGAO, Ademar Benevolo, 13213-009 JUNDIAI - SP (BR); GOMEZ, Hernan Cortes, 13213-009 JUNDIAI - SP (BR); SCAVONE, Raphael Gomes, 13213-009 JUNDIAI - SP (BR)
(74) Representative: Potter Clarkson
(86) International application number: PCT/BR2022/050166
(87) International publication number: WO 2022/236392

(56) References cited:
- EP-B1- 2 836 266
- WO-A1-2014/167597
- CN-A- 112 280 091
- DE-A1- 102005 042 181
- US-A1- 2021 331 107
- LA SPINA RITA ET AL: "Synthesis of Citrate-Stabilized Silver Nanoparticles Modified by Thermal and pH Preconditioned Tannic Acid", NANOMATERIALS, vol. 10, no. 10, 15 October 2020 (2020-10-15), pages 2031, XP093261698, ISSN: 2079-4991, DOI: 10.3390/nano10102031
- ROSA PAULA DE FREITAS; AGUIAR M?NICA LOPES; BERNARDO ANDR?: "Modification of Cotton Fabrics with Silver Nanoparticles for Use in Conditioner Air to Minimize the Bioaerosol Concentration in Indoor Environments", WATER, AIR,, SPRINGER INTERNATIONAL PUBLISHING, CHAM, vol. 228, no. 7, 14 June 2017 (2017-06-14), Cham, pages 1 - 9, XP036274641, DOI: 10.1007/s11270-017-3429-y
- SOARES DA SILVA MOÇO SALOMÉ: "Impregnação de fibras de celulose com nanopartículas de prata, óxido de zinco e óxido de cobre para aplicações antibacterianas", DISSERTAÇÃO, GRUPO DE INVESTIGAÇÃO FACES DE EVA, ESTUDOS SOBRE A MULHER DA FACULDADE DE CIÊNCIAS SOCIAIS E HUMANAS DA UNIVERSIDADE NOVA DE LISBOA, 1 November 2013 (2013-11-01), XP093007063, [retrieved on 20221212]
- ROSA PAULA DE FREITAS; AGUIAR MôNICA LOPES; BERNARDO ANDRé: "Modification of Cotton Fabrics with Silver Nanoparticles for Use in Conditioner Air to Minimize the Bioaerosol Concentration in Indoor Environments", WATER, AIR,, SPRINGER INTERNATIONAL PUBLISHING, CHAM, vol. 228, no. 7, 14 June 2017 (2017-06-14), Cham, pages 1 - 9, XP036274641, ISSN: 0049-6979, DOI: 10.1007/s11270-017-3429-y
- JINGA S I, ISOPENCU G, STOICA-GUZUN A, STROESCU M, FERDES M, OHREAC B: "SILVER GREEN SYNTHESIS ON BACTERIAL CELLULOSE MEMBRANES USING TANNIC ACID", DIGEST JOURNAL OF NANOMATERIALS AND BIOSTRUCTURES, vol. 8, no. 4, 1 October 2013 (2013-10-01), pages 1711 - 1717, XP093007067

## Description

### FIELD OF INVENTION

The present invention relates to a heat and moisture exchange filter that provides antimicrobial bioactivity (i.e., antibacterial, antifungal and antiviral activity) for use in mechanical respiration.

### DESCRIPTION OF THE PRIOR ART

When patients undergo mechanical respiration, heating and humidification of inspired gases are essential.

Heat and Moisture Exchangers (HME - Heat and Moisture Exchanger) are one of the device options currently available to perform these functions.

Some examples of HMEs are described in document US867391, which describes a device that was used to maintain the humidity and temperature of a gas aspirated for a patient to inhale under anesthesia; and in document EP2113278, which discloses an HME heat enthalpy that is suitable for use in anesthesiology and intensive care, to passively heat and moisten the respiratory gas supply to the patient.

The devices described in documents DE102014005241 and US6209541 feature a particle filtering membrane. Document DE102014005241 describes an effective HME filter that is stable over time and has reduced complexity. Document US6209541 deals with a hydrophobic electrostatic respiratory filter used as a barrier to particulate matter.

It is assumed that all types of HMEs trap some amount of water vapor in the expiratory (exhaled) gas at condensation points between the spaces formed in an element of the HMEs. The suction gas is used to evaporate condensed water for humidification. In case of heating of the inhalation gas, it is expected that cyclical phenomena and local thermal storage will occur. Another problem is related to clogging or encrustation caused by the accumulation of condensed water and secretions that obstruct the patient's airways, resulting in asphyxiation.

These humidified gases are vectors of bacterial, fungal and viral transfer and can contaminate mechanical breathing equipment. Therefore, there is a risk of cross-contamination of equipment components by microorganisms transmitted by saliva and also by exhaled gas.

In addition, common HMEs have filters with polymeric fiber materials in layers arranged in a sandwich style. These fibers have many disadvantages, including ineffectiveness in filtering submicrometer particles (e.g., viral particles vary in diameter from 60 to 140 nm), which can easily cause fouling in the fiber, restricting airflow. Particulate matter with dimensions less than 2.5 µm is generally considered to be more harmful to human health due to its ability to penetrate the human respiratory system and the ability to deposit in the bronchi and lungs, which is seen in the case of pathogens (Ali et al. 2018).

Therefore, these filters become uneconomical and ineffective against small particles. Furthermore, conventional filters which are known to have low efficiency air filtration performance and therefore suffer from durability resulting in low effectiveness.

These facts demonstrate the importance of the presence of a biocidal system in HMEs, considering that the absence of an effective antimicrobial system allows bacterial, fungal and viral deposition in the equipment, making it a potential source of contamination and infection.

Nosocomial infections lead to a series of serious clinical complications in hospital settings. This inconvenience and expense puts the standard of patient care at risk. The current environment produces the emergence of bacterial, fungal and viral strains with multi-resistance to antimicrobials.

Furthermore, the development of a filter that exhibits high filtration capacity for micrometer and submicrometer particles is also essential.

There is a need to develop HME filters with high stability, durability, cost-effectiveness and effective antibacterial, antifungal and antiviral properties.

Thus, given the reported drawbacks, the present invention aims to provide a heat and moisture exchange filter with biocidal activity that is capable of immobilizing and eliminating/inactivating microorganisms and viral particles.
WO 2014/167597 A1 discusses a filter sheet for preventing airborne dust, particles, pollen, viruses, bacteria and harmful gases from entering the body through the mouth and nose, and in particular to a multi-layer fiber filter sheet that has an antibacterial, antifungal, antiviral, deodorizing, moisturizing and heat-retaining properties and is designed to be inserted into a mask or attached to a filter machine or device.

EP 2,836,266 B1 discusses a mounting for speech valves and/or heat-moisture exchangers and heat-moisture exchangers suitable therefore, which are preferably used with tracheostomy dressings.

CN 112,280,091 A discusses a biomass-based antiviral filtering material and a synthetic method thereof, the biomass-based antiviral filtering material comprises wood powder nano-fibril.

DE 10 2005 042181 A1 discusses plastics for medical devices and relates to molded bodies for forming medical devices and to processes for producing plastics and/or molded bodies according to the invention.

US 2021/331107 A1 discusses a filtration article for use in personal protective equipment that includes one or more layers.

### BRIEF DESCRIPTION OF THE INVENTION

The invention is defined in the appended claims.

The present invention deals with a heat and moisture exchange filter with biocidal activity that comprises polymeric cellulose fibers impregnated with silver nanoparticles (AgNPs), wherein the silver nanoparticles comprise a mixture of silver nanoparticles obtained from tannic acid and silver nanoparticles obtained from sodium citrate, wherein the silver nanoparticles obtained from tannic acid have an average size obtained by transmission electron microscopy (TEM) ranging between 10 and 30 nm; and the silver nanoparticles obtained from sodium citrate have an average size obtained by transmission electron microscopy (TEM) ranging between 30 and 50 nm.

The present invention also deals with a method of preparing a heat and moisture exchange filter with biocidal activity that comprises the following steps:
a) Preparation of silver nanoparticles with tannic acid;
b) Preparation of silver nanoparticles with sodium citrate;
c)Mixing the nanoparticles obtained in steps (a) and (b) in a proportion ranging between 1:4 and 4:1;
d) Impregnation of polymeric cellulose fibers with the mixture of silver nanoparticles obtained in step (c); It is
e) Drying the impregnated cellulose fiber obtained in step (d).

The invention described here also refers to the use of silver nanoparticles that are impregnated into polymeric cellulose fibers to produce a heat and moisture exchange filter with biocidal activity.

### BRIEF DESCRIPTION OF FIGURES

For a better understanding of this disclosure, a detailed description is given below, making references to the attached drawings:
FIGURE 1A is an example of a complete integrated heat and moisture exchanger system;
FIGURE 1B shows a diagram of how the air that passes through the mechanical fan is purified;
FIGURES 2A and 2B are nanometer-scale images obtained by transmission electron microscopy (TEM) of the nanoparticles obtained by tannic acid (TA-AgNPs) and sodium citrate (CT-AgNPs) and the respective size distribution histograms, and in the figure 2A, the results obtained with TA-AgNPs are represented, while in figure 2B, the results obtained with CT-AgNPs are represented;
FIGURE 3 shows the heat and moisture exchange filter of the present disclosure with the ability to inactivate pathogens through a Trap Zap system;
FIGURE 4 is a graph showing the H2O absorption and retention capacity of the HME in relation to the amount of saturated vapor in the air at 37°C (normal body temperature);
FIGURE 5 diagram of preparation of silver nanoparticles and methods of impregnation on cellulose fibers to form the heat and moisture exchange filter of the present invention;
FIGURE 6 are images obtained by scanning electron microscopy (SEM) of cellulosic fibers impregnated with AgNPs and qualitative evaluation of Ag based on analysis of energy dispersive spectroscopy (EDS);
FIGURE 7 shows representative views of the geometric and topographic properties of the heat and moisture exchange filter of the present disclosure, indicating the mechanism of action attributed to a biocatalytic surface with the propensity to release Ag+ ions; It is
FIGURE 8 are graphs showing the tensile stress of examples of heat and moisture exchange filters of the present invention with improved shear strength.

### DETAILED DESCRIPTION OF THE DISCLOSURE

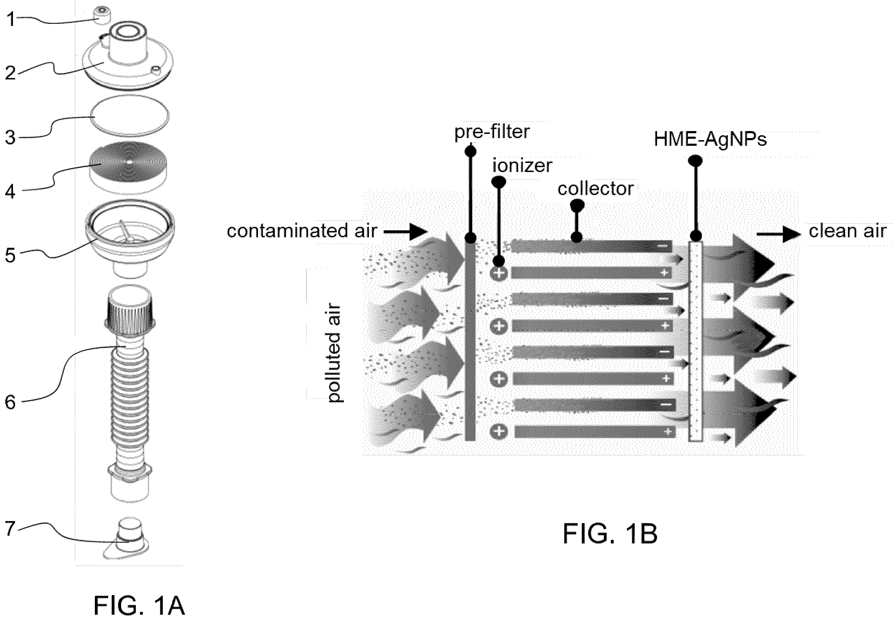

The present disclosure, as shown in figure 1A, refers to a heat and moisture exchanger filter (HME - Heat and Moisture Exchanger) with biocidal activity that is located between the electrostatic filter membrane (3) and the lower body ( 5).

In Figure 1A, a common example of a complete integrated heat and moisture exchanger system used in mechanical fans is presented, being: cover (1), top cover (2), electrostatic filtering membrane (3), heat exchanger filter and moisture with biocidal activity (4), lower body (5); extendable corrugated trachea (6), dual-purpose trachea cover (7).

Figure 1B shows a diagram of how the air that passes through the mechanical fan is purified using the heat and humidity exchanger with biocidal activity of the present disclosure. After passing through the pre-filter, ionizer and collector, the unsuitable air loaded with microorganisms and viral particles completes its purification by passing through the heat and moisture exchange filter with biocidal activity (represented in the figure by the acronym HME-AgNPs).

The heat and moisture exchange filter with biocidal activity - HME comprises polymeric fibers
in cellulose impregnated with nanoparticles
in silver (AgNPs) displayingantimicrobial properties against bacteria, fungi and viruses.

Cellulose polymer fiber is widely used in the manufacture of textiles, for example: clothing, feminine hygiene products, medical-surgical products, filters Disposable respirators designed and configured to suit most respiratory and other applications.

For use in the present disclosure, the surfaces of the cellulose fibers are preferably dry and clean of any oily or paraffinic residue.

According to the disclosure, the cellulose fiber layer of the heat and moisture exchange filter is preferably in the form of a double partitioned layer.

The heat and moisture exchange filter according to the present disclosure comprises cellulose with a small amount of calcium chloride, which acts as a heat storage carrier material in the range of 20-80 kg/m3. Other hygroscopic salts to aid in the absorption of H2O from the air can be used, including sodium hydroxide and magnesium chloride. However, the use of calcium chloride is preferred considering that this component is non-toxic and is generally recognized as safe (GRAS - designation from the Food and Drug Administration (FDA), United States).

The use of cellulose as a polymeric material and AgNPs as a metal dopant provides a useful nanocomposite material due to its robust renewable nature, biodegradability It is antimicrobian activity.

According to the present disclosure, silver nanoparticles (AgNPs) are present on the surface of the cellulosic material or immersed in it.

The AgNPs incorporated into the exposed cellulose fibers and the surface fibers result in a catalytically active surface and wherein said AgNPs exhibit a highly controlled propensity for Ag+ ion release when exposed to or in close proximity to water or water vapor containing microbial particles ( bacteria, fungi and yeast).

According to the present disclosure, the heat and moisture exchange filter with biocidal activity comprises silver nanoparticles obtained by reducing agents chosen from tannic acid, sodium citrate, chitosan, ascorbic acid, thiosulfate, polyethylene glycol or mixtures thereof. The present invention uses silver nanoparticles obtained from tannic acid and sodium citrate.

Other examples of reducing agents that can be used in the absence, addition or replacement of sodium citrate and tannic acid without increasing toxicity, without generating toxic residues or by-products and without the effective loss of antimicrobial and antiviral properties are phytochemical reducers, such as: mangiferin catechins derived from Camellia sinensis, resveratrol, pomegranate extract, ascorbic acid, Syzygium cumini extract, Annona muricata extract, Plinia cauliflora extract etc.

In the absence or unavailability of raw materials on the market (phytochemical and reducing agents of natural origin such as: polyols, polyphenols, flavonoids, polysaccharides and proteins) to synthesize nanoparticles using green chemistry routes, it is possible to use conventional chemical reducing agents to meet market demand. Examples of chemical reductants are hydrazine, sodium tetraborohydride (NaBH4), other reductants include active metals, such as sodium, magnesium, aluminum and zinc, which have relatively small ionization energies and low electronegativities. Metal hydrides, such as NaH, CaH2 and LiAlH4, which formally contain the H- ion, are also good reducing agents. It is still possible to use physical methods to produce silver nanoparticles such as microwaves, ultrasound, x-rays and gamma radiation.

The average size of AgNPs obtained by transmission electron microscopy (TEM) is between ~10 and 55 nm. Preferably, AgNPs with particle size <20 nm are used, as this presents the best antimicrobial activity, due to the surface-to-volume relationship for the propensity and ease of release of Ag+ ions. The size of the produced AgNPs is adjustable with an average size of 17 nm.

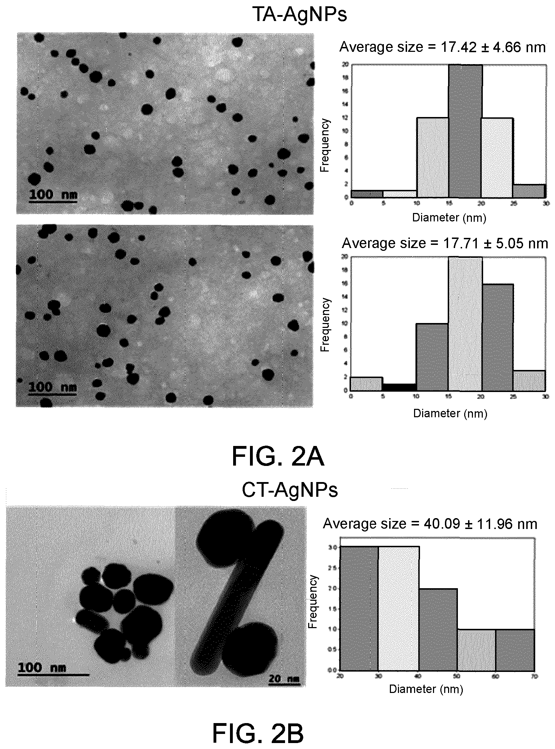

Figures 2A and 2B show the images obtained by TEM of the silver nanoparticles obtained using tannic acid (TA-AgNPs) with an average size of 17.71 ± 5.05 nm (Figure 2A) and the silver nanoparticles obtained with the sodium citrate (CT-AgNPs) with an average size of 40.09 ± 11.96 nm (Figure 2B).

The size ranges of the nanoparticles obtained by dynamic light scattering (DLS) of the two nanoparticles are 5 to 90 nm. It is worth mentioning that both preparation methodologies allow obtaining AgNPs of controlled size (1 to 100 nm), as long as the synthesis parameters are adjusted, such as the concentration of reagents, temperature, pressure and agitation.

The present invention include a synergy between AgNPs produced by sodium citrate (CT-AgNPs) and tannic acid (TA-AgNPs) that provides improved antimicrobial efficacy. The synergy between TA-AgNPs and CT-AgNPs is attributed to the size and shape of the AgNPs, where CT-AgNPs are also composed of rod-shaped AgNPs, which are all an important factor contributing to the degree and potency of cytotoxicity.

The cellulosic fibers of the heat and moisture exchanger have -OH groups on the periphery of the monosaccharide structure, in addition to sodium citrate and tannic acid containing oxygen atoms and aromatic structures, respectively, which allow the complexation of silver nanoparticles and the formation of cellulose-O-Ag type bridge. The binding of AgNPs to cellulose is also encompassed by electrostatic forces, complexation phenomena, Van der Waals, hydrogen bonds or π-π stacking permanently linked to it (Li et al., 2019).

Another aspect of the present disclosure provides increased density, surface area, piercing rate and number of cells of the heat storage carrier material that constitutes the respiratory heat and moisture exchanger.

Thus, the heat and moisture exchanger filter with biocidal activity of the present disclosure presents a catalytically active surface with AgNPs and in which said AgNPs exhibit a highly controlled propensity to release Ag+ ions when exposed to or in close proximity to water or water vapor. containing microbial particles (i.e. viral particles), respectively.

The high spatial arrangement of AgNPs (<10 nm) in the morphological and interfacial characteristics of the combination with cellulosic fiber material, silver nanoparticles bind through the sulfur-containing residues of gp120 glycoprotein receptors expressed on the membrane of pathogenic microorganisms. The high specificity of AgNPs in their natural tendency for disulfide bonds plays an important role in antimicrobial activity, therefore, blocking the binding of pathogenic microorganism with subsequent inactivation through Trap Zap technology. In the Trap Zap system, the textured surface topography with AgNPs provides synergy through: I) upon adhesion of the microbial surface, the textured surface traps ("Trap") the microbes upon binding to the filter surface due to the presence of air trapped between topographies; and II) the presence of AgNPs releases Ag+ ions, resulting in lipid peroxidation ("Zap") of the phospholipid layer present in the outer membrane of microbes, which leads to cellular damage.

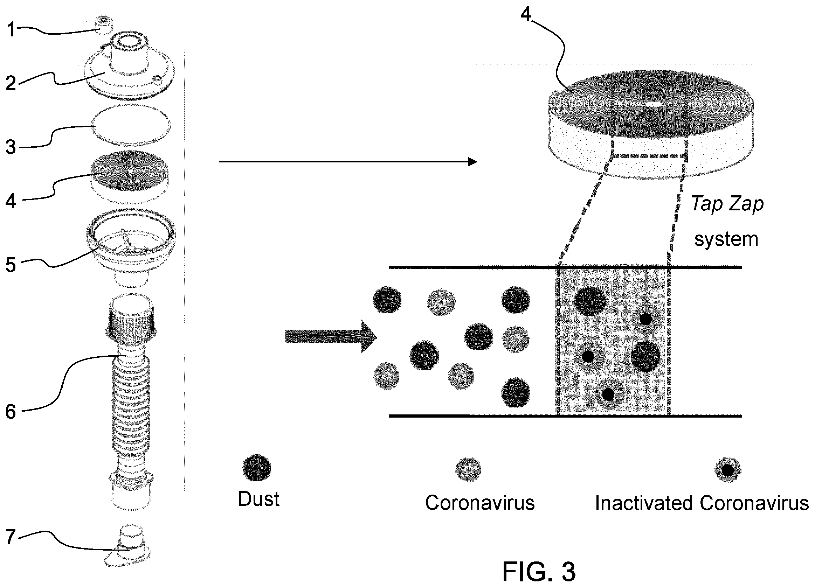

Figure 3 presents an example diagram of the Trap Zap system acting in air laden with dust and coronavirus. When passing through the heat and moisture exchange filter with biocidal activity of the present disclosure, the dust particles are retained in the filter and the coronavirus is inactivated by the presence of AgNPs.

Therefore, the texture surface topography with AgNPs provides synergy through: I) the surface texture reduces the adhesion of the virus to the filter surface due to the presence of air trapped between the topographies; and II) the presence of release of Ag+ ions from AgNPs results in lipid peroxidation of the phospholipid layer present in the external membrane of the pathogens.

Particularly, the surface of the heat and moisture exchange filter must be highly corrugated to avoid pressure loss.

The air flow considered over time can humidify the gas to 100%, therefore, the exhaled human air is completely saturated with water, thus containing maximum amount of moisture (RH=100%), passing through the heat and humidity exchanger filter of the present disclosure eliminates or inactivates approximately 99.9% of pathogenic microbial particulates.

One of the anticipated aspects of the present disclosure is that silver nanoparticles would be expected to dissolve in solution. However, surprisingly, this did not happen.

In this way, the present disclosure provides sufficient durability and effectiveness, making it safe and affordable for consumers against pathogenic microorganisms, nosocomial infection and other infectious diseases.

Although silver nanoparticles are stable when produced, optionally, an additional stabilizing agent chosen from polyvinylpyrrolidone (PVP), polyvinyl alcohol, xanthan gum, gum arabic, guar gum, starch and its derivatives, cellulosic derivatives (carboxymethyl cellulose (CMC), EMC) is used. etc.), starches (potato, cassava, etc.), agar or mixtures thereof. Preferably, polyvinylpyrrolidone (PVP) is used as an additional stabilizer.

PVP was the polymer chosen to formulate the AgNPs of the present disclosure, due to its stabilizing and adhesive properties. In order to promote increased stability of AgNP suspensions and add adhesive capacity for the incorporation of AgNPs into the heat and moisture exchange filter with biocidal activity of the present disclosure.

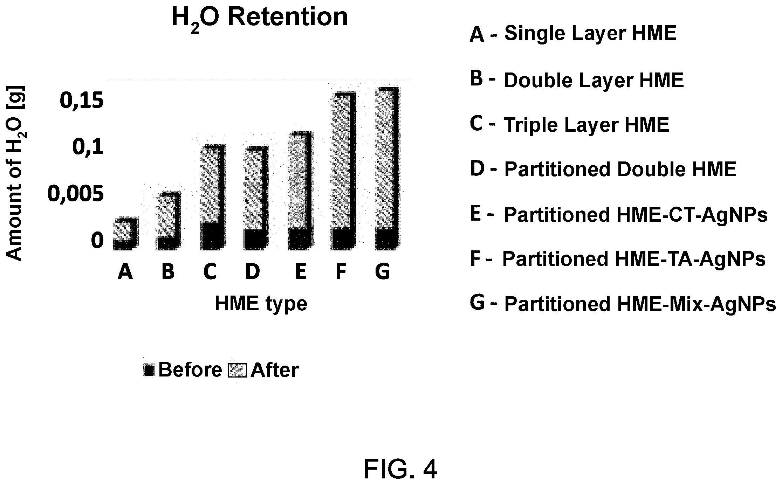

Additionally, the present disclosure increased the water retention and storage capacity. As shown in Figure 4, an amount of saturated vapor in air at 37°C (normal body temperature) maintains high absolute humidity recovery. The incorporation of AgNPs increased tear strength with increased tensile strength, hydrophilicity, and overall strong mechanical stress of the cellulose filter that increased static water retention and dynamic water retention. Furthermore, as PVP provided stability to AgNPs, it also functions as a semi-interpenetrating polymer network, providing robust water retention capacity.

Figure 4 also shows the hydrophilicity attributed to the adhesive strength and antimicrobial properties of AgNPs. Thereafter, said components impart a hygroscopic property to the heat and moisture exchanger filter of the present disclosure which is enhanced through increased H2O retention capacity and provides a barrier to droplets and particles that potentially carry pathogens. In Figure 4, there is: A - HME containing a single layer of cellulose fiber; B - HME contains two layers of cellulose fiber pressed together; C - HME contains three layers of cellulose fiber sandwiched together; D - HME containing two layers of cellulose fiber with standardized geometric and topographic modification sandwiched together; E - HME containing two layers of cellulose fiber with CT-AgNPs and standardized geometric and topographic modification sandwiched together; F - HME containing two layers of cellulose fiber with TA-AgNPs and standardized geometric and topographic modification sandwiched together; and G - HME containing two layers of cellulose fiber with a mixture of AgNPs (TA-AgNPs and CT-AgNPs) and sandwiched standardized geometric and topographic modification.

In one embodiment of the disclosure, the heat and moisture exchange filter with biocidal activity of the present disclosure comprises biocompatible silver nanoparticles, stabilized with material based on tannins or phytochemicals rich in polyphenols and, specifically, with tannic acid (TA), a specific form of tannin. Tannic acid may be from a commercial source (e.g., Sigma Aldrich, St. Louis, Mo., USA), or it may be derived from any of the following plant parts: Tara pods (Caesalpinia spinosa), Rhussemialata nuts or Quercus infectoria or leaves of Sicilian sumac (Rhuscoriaria). The silver nanoparticles of the disclosure can be manufactured with an eco-frendly method, based on the principles of Green Chemistry, to make stable and biocompatible silver nanoparticles.

The manufacturing methods of the disclosure require only silver nitrate salt as a precursor. No other synthetic chemicals are employed in the overall manufacturing process and therefore there are no harsh chemicals used in manufacturing or residual by-products formed during manufacturing. The manufacturing processes of the disclosure are therefore environmentally friendly and biologically effective (benign).

Preferred embodiments provide an environmentally friendly nanotechnology route for producing silver nanoparticles. The methods of the disclosure use tannic acid as an electron reservoir and a reducing agent, to reduce the silver salt to the corresponding silver nanoparticles in the absence of any other reducing agent and without any harsh chemicals. A preferred process for forming silver nanoparticles is to react the silver salt with tannic acid. No toxic chemical reducing agents are used, and the methods avoid any use of toxic agents such as hydrazine, sodium borohydride, etc. A preferred embodiment is simply to mix the purified tannic acid compound with nitrate salt in water at suitable concentrations and reaction conditions.

Silver nanoparticles obtained from tannic acid (TA-AgNPs) can be produced in a preferred size range of ~10 nm to 30 nm. The size range was determined by obtaining transmission electron microscopy (TEM) images as shown in Figure 2. After filtering, the TA-AgNPs nanoparticles did not precipitate and remained stable in solution. TA-AgNPs were produced with high negative Zeta potential in the range of -24 to -41 mV (millivolts), suggesting excellent stability of these nanomaterials due to electrostatic repulsion.

Example experiments synthesized silver nanoparticles by reduction with tannic acid in water. The measurements were used to characterize the particles formed. The experiments showed that tannic acid also plays a unique role in not only reducing the AgNO3 salt but also stabilizing the reduced silver atoms to form nanoparticles and maintain the monodisperse nature of the suspension. Tannic acid rings are active and have abundant hydroxyl groups present in the aromatic structure of polyphenols that play a significant role in reducing silver nitrate to silver atoms.

No additional treatment is necessary before using the silver nanoparticles produced by this method, as they are stable and biocompatible for biomedical applications. The method produces biocompatible, stabilized silver nanoparticles that are suitable for use inside the body (in vivo) for diagnostic and treatment procedures. The biocompatible and stabilized silver nanoparticles of the disclosure do not require further purification and are suitable for direct administration into the human body orally or intravenously.

In another embodiment of the disclosure, the heat and moisture exchange filter with biocidal activity comprises silver nanoparticles obtained from the adapted classical method, in which citrate (CT) was used as a reducing and stabilizing agent. As in the method described with tannic acid (TA), the concentration of reagents and reaction conditions can be varied to obtain sizes and shapes of nanoparticles suitable for the desired application.

CT-AgNPs were synthesized with sizes greater than 30 nm (between 30 and 50 nm by TEM) . After filtration, the CT-AgNPs did not precipitate and remained stable in solution. Therefore, the average size (40.09 ± 11.96) was determined by obtaining transmission electron microscopy (TEM) images as shown in Figure 2.

The CT-AgNPs were produced with high negative Zeta potential in the range of -28 to -44 mV (millivolts), suggesting excellent stability of these nanomaterials due to electrostatic repulsion.

The adaptation of the method using citrate as a reducing and stabilizing agent was made in order to make the process ecologically viable, which does not require the use of toxic chemical reduction agents. Thus, the methods avoid any use of toxic agents, such as hydrazine, sodium borohydride, etc. The manufacturing method of the disclosure requires only silver nitrate salt as a precursor. No other synthetic chemicals are employed in the overall manufacturing process and therefore there are no harsh chemicals used in manufacturing or residual by-products formed during the process.

Sodium citrate is produced by completely neutralizing citric acid with a high-purity sodium source such as sodium hydroxide, sodium bicarbonate or sodium carbonate. Sodium citrate comes in the form of a white or crystalline powder, very soluble in water and practically insoluble in alcohol. Citric acid is present in many acidic fruits and foods. It also makes up one of the main energy cycles in eukaryotic cells, called the citric acid cycle, better known as the Krebs cycle.

In another embodiment of the **disclosure,** the heat and moisture exchange filter with biocidal activity comprises a mixture of silver nanoparticles obtained from tannic acid and calcium citrate, in order to increase the spectrum of action of AgNPs against various microorganisms, taking into account the different sizes of AgNPs.

For incorporation into the biocidal system of the present disclosure, a mixture of ATAgNPs with CT-AgNPs was prepared in order to obtain a diversity of nanoparticles in terms of size, shape and charges. The ratio between TA-AgNPs and CT-AgNPs ranges from 1:4 to 4:1.

The present disclosure also relates to the method of preparing the heat exchanger filter and moisture with biocidal activity that comprises the following steps:
a) Preparation of silver nanoparticles with tannic acid;
b) Preparation of silver nanoparticles with sodium citrate;
c) Mixing the nanoparticles obtained in steps (a) and (b) in a proportion ranging between 1:4 and 4:1;
d) Impregnation of polymeric cellulose fibers with the mixture of silver nanoparticles obtained in step (c); It is
e) Drying the impregnated cellulose fiber obtained in step (d).

Particularly, step (d) of impregnation of cellulose polymer fibers with the mixture of silver nanoparticles occurs by spraying or immersion.

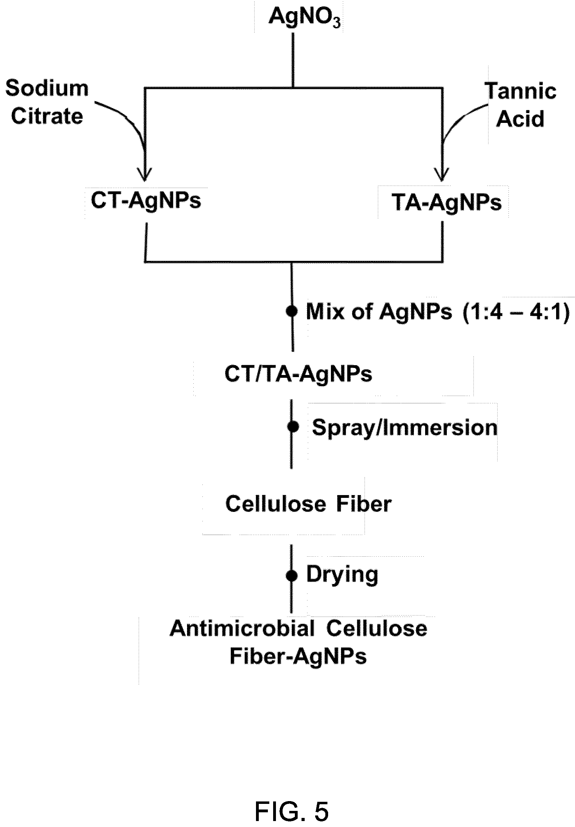

Figure 5 illustrates in a simplified way the steps for preparing the silver nanoparticles and the impregnation methods in the biocidal system of the present disclosure.

The disclosure also deals with the use of silver nanoparticles that are impregnated in polymeric cellulose fibers to produce a heat and moisture exchange filter with biocidal activity.

### EXAMPLES

In the experimental phase described below, the reagents: Silver Nitrate, Tannic Acid (TA), Sodium Citrate (CT) and polyvinylpyrrolidone (PVP), were obtained from Sigma Aldrich, St. Louis, Mo., USA . The silver nanoparticles were obtained based on green chemistry and green nanotechnology methods, which minimize the use of toxic reagents to living beings and the environment, aiming at the concept of sustainability.

### Production of heat and moisture exchange filter

The preparation of the heat and humidity exchanger filter comprises the following steps:
**Step 1-** production of silver nanoparticles with tannic acid (TA-AgNPs) - the AT solution was prepared in different concentrations (0.5 to 3 mM) with PVP solution (0.1 to 2%) to which the solution is added of silver nitrate (0.3 to 2 mM) in a ratio of 1:4 (v/v) at room temperature. The solution was stirred for 30 minutes and the spectral data of the synthesis were collected;
**Step 2-** production of silver nanoparticles with citrate (CT-AgNPs) - the citrate solution (CT) was prepared in different concentrations (05 to 5%) with PVP solution (0.1 to 2%) to which the solution was added of silver nitrate (0.3 to 2 mM) in a ratio of 9:10 (v/v) at 100°C. After 15 minutes of reaction, heating was stopped and the solution remained stirring until it reached room temperature. Next, the spectral data of the synthesis were collected; It is
**Step 3-** impregnation of cellulosic fiber - a mixture of silver nanoparticles was prepared with tannic acid (TA-AgNPs) and calcium citrate (CT-AgNPs), in a proportion ranging between 1:4 and 4:1.

Then, the cellulosic fiber was impregnated with a mixture of TA-AgNPs and CT-AgNPs using a pneumatic R12 standard spray gun JET JAT-500 HVLP approx. airflow rate at ~18 to 25 psi with 150mm / 5.5 spray distance.

After drying the cellulose fiber impregnated with the mixture of TA-AgNPs and CT-AgNPs, a heat and moisture exchange filter is obtained.

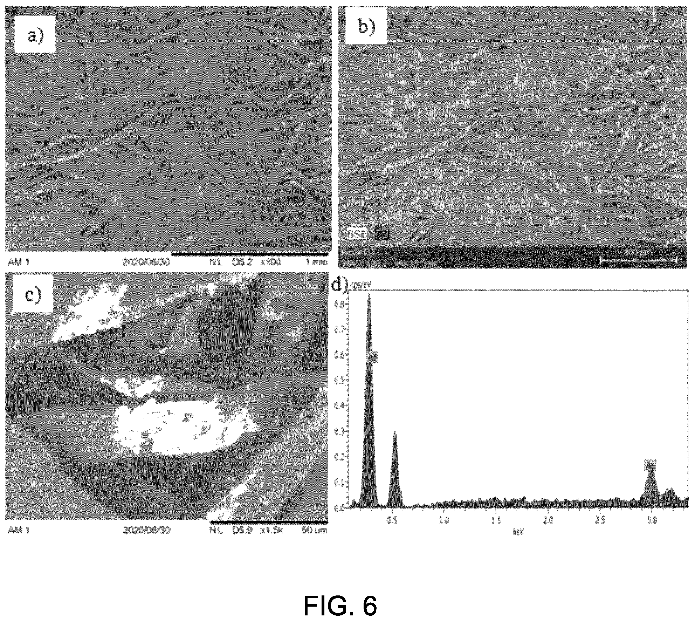

As can be seen in Figure 6, which presents a scanning electron microscope (SEM) photograph and energy dispersive X-ray spectroscopy (EDS) map of the cellulose filter impregnated with TA-AgNPs, the embedded silver nanoparticles project up from the surface fibers and result in a catalytically active surface (Scale bar, bottom right = 200 micrometers (µm)). In Figure 6, there is: a) SEM magnification at x100, b) EDS map, c) magnification at x 1.5k and d) Spectrum of the EDS map.

### Measurements and Characterization

Spectroscopic measurements were performed on a Luminescence multimode SpectraMax i3x microplate reader (Molecular Devices, LLC, San Jose, CA, USA) and disposable cuvettes with a volume of 1 mL and a path length of 10 mm. The hydrodynamic diameter and Zeta potential were obtained using the Zetasizer Nano S90 (Malvern Instruments Ltd. USA). Transmission Electron Microscope (TEM) images were obtained on a JEOL 1400 TEM (JEOL, LTE, Tokyo, Japan).

### Cutting and processing

The present disclosure provides a geometric and topographic property to cellulosic fiber manufactured with silver nanoparticles. Fiber that is passed through a rotary cutter can be processed to have improved mechanical properties in relation to its mechanism of action and applications.

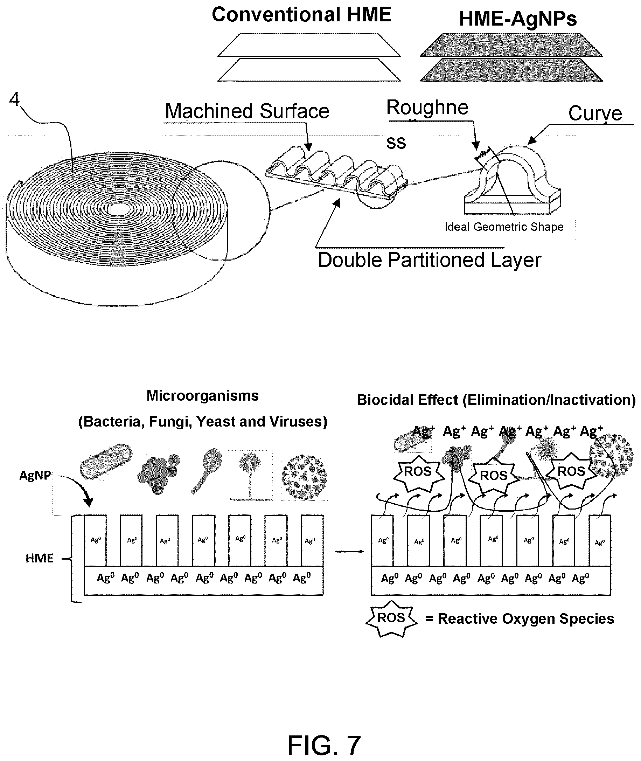

Figure 7 shows the mechanism of microbicidal and antiviral activities of the HME-AgNPs heat and moisture exchanger filter developed to exhibit strong microbicidal activity against microorganisms/viruses via reactive oxygen species (ROS) and silver ions on the surface of the substrate. It is imperative to also consider the geometry and topographical properties of cellulose fiber impregnated with AgNPs in relation to their mechanism of action. Surface texture, roughness, and chemistry are critical to the effectiveness of bioactive HME.

Furthermore, the surface markedly influences the surface tension between microorganisms (i.e., bacteria, fungi, viruses, and other pathogens) and the HME surface, including the propensity to release Ag+ ions, increased bearing area, wear and tear resistance. , therefore increasing tensile strength. The topography of HME-AgNPs can decrease the adhesion of the virus on the surface due to the presence of AgNPs that release Ag+ ions resulting in lipid peroxidation of the phospholipid layer present on the outer envelope of the test pathogens.

The toxicity of AgNPs is attributed to the propensity to release silver (Ag+) ions from AgNPs and their soluble complexes (Zhou et al., 2016) and maintain a constant flow of a high concentration of Ag+ ions from AgNPs, which is crucial for the antimicrobial activity as shown in Figure 7.

The AgNPs on the exposed surface are dissolved and oxidized on the highly moist surface which leads to the formation of Ag+ ions. After that, Ag+ ions diffuse into the absorbed water layer containing the pathogens of the parent particles (AgNPs). The hypothesis is that the airflow considering the respiratory system can humidify the gas to 80-90%, therefore, exhaled human air is completely saturated with water, thus containing the maximum amount of moisture (RH = 100%), passing The heat and moisture exchange filter of the present disclosure eliminates or inactivates approximately 99.9% of microbial particles.

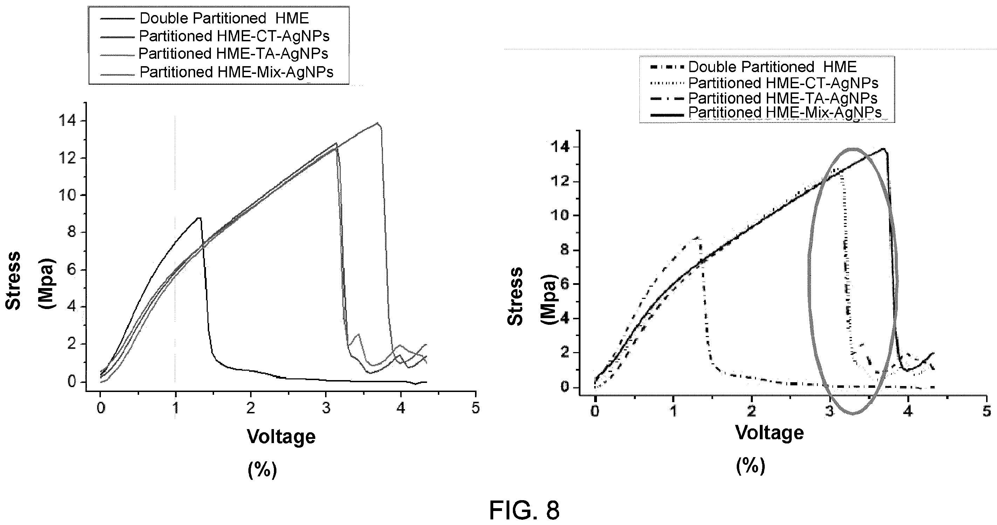

The texture, roughness and chemistry of the surface are fundamental to the effectiveness of the heat and moisture exchange filter of the present disclosure. Furthermore, the surface predominantly influences the surface tension between the microorganism and the catalytic surface of the cellulosic fiber incorporated with silver nanoparticles, including silver binding affinity and load capacity, increased support area, tear resistance with increased tear resistance. traction and tension (Figure 8).

### Biological activity

Cellulosic fibers with silver nanoparticles exposed and projected from the surface of the fibers result in a catalytically active surface and said silver nanoparticles exhibit a highly controlled propensity to release Ag+ ions when exposed to or in close proximity to water or water vapor containing microbial particles or viral particles. Biological activity can be demonstrated using routine assays, including, but not limited to, those described in patent US7169402. Suitable assays include the AATCC 147-2016 test method and the viral inhibition test in the aforementioned patent.

### Antibacterial and antifungal investigations

### AATCC 147-2016 Test Method

Test microorganisms:
*I. Staphylococcus aureusATCC* No. 6538,
*II. Klebsiella pneumoniaeATCC* No. 4352,
*III. Candida albicansATCC* 10231, and
*IV. Aspergillus brasiliensisATCC* 16404 Viral inhibition of nanoparticles from respirator-treated prototypes

10-day-old specific pathogen-free (SPF) chicken embryos were used to titrate Gammacoronavirus (Gamma-CoV) and an infectious chicken bronchitis virus, which is an enveloped virus. The Massachusetts strain was used as no Gamma-CoV specific antibody can interfere with GammaCoV replication, thus ensuring the validity of the results by serving as a quality assurance/control measure.

### GammaCoronavirus titer in fertilized eggs by EID50

Ovoscopy (Avifag Comercial, Rio Claro/SP, Brazil) was used to visualize the egg compartments. A mark of the inoculation site was made on the edge of the air bag. Gamma-CoV titer was serially diluted in diluent medium (2.5% w/v tryptose, 1,000 U/mL penicillin, 1,000 ug/mL streptomycin). Eggshells were disinfected with 70% ethanol and allowed to dry, followed by a small hole or perforation in the eggshell at the inoculation site under aseptic conditions.

The eggs (n = 10) by dilution, were inoculated with 0.2 mL of Gamma-CoV in the allantoic cavity by inserting the 25 G needle approx. 16 mm in the egg.

After inoculation, the hole in each egg was sealed with melted wax and the eggs were incubated in an egg incubator at 37.8°C with 60% RH. After 24 hours, an ovoscopy was performed and discarded. you embryos withnonspecific mortalities. Were realizedlight ovoscopies every 2 days. After 10 days of incubation, the eggs were analyzed for embryodiagnosis and the ED50 was calculated. The slow filtration methodology was used in prototype respirators treated with nanoparticles. Viral titration and analysis were performed according to Kint et al. (2015) with some adaptations.

In view of the experiments carried out, we believe that the heat and humidity exchanger filter of the present disclosure brings several advantages in relation to state-of-the-art devices. The following advantages stand out:
The spray and immersion technique for impregnating silver nanoparticles is instantaneous, with short reaction and contact times. Surprisingly, exposure to a silver ion did not affect the chemical and structural characteristics of said fiber. This provides a method to optimize HME's heat storage capacity, antibacterial, antifungal and antiviral properties.

The thermal storage capacity of the heat and moisture exchanger filter with biocidal activity must be improved to maintain the required temperature and stability attributed by increased traction, stress resistance and thermal resistance.

The heat and moisture exchanger filter with biocidal activity of the present invention is compatible with final nebulization of the patient. There is no entrainment or release of AgNPs in the breathing/ventilation system.

It will be apparent to those skilled in the art that although the invention is not limited to the details of the foregoing illustrative examples and that the present invention may be embodied in other specific forms without departing from the essential attributes thereof, and therefore it is desired that the present embodiments and examples are considered in all respects as illustrative and not restrictive, reference being made to the appended claims rather than to the foregoing description, and all changes that come within the meaning and range of equivalence of the claims are therefore intended to be indexed to the request. Bibliographic references
Ali, A., Pan, M., Tilly, TB., Zia, M., Wu, CY. (2018). Performance of silver, zinc, and iron nanoparticles-doped cotton filters against airborne E. coli to minimize bioaerosol exposure. Air Quality, Atmosphere & Health. 11:1233-1242https://doi.org/10.1007/s11869-018-0622-0 Li, J., Liu, X., Tomaskovic-Crook, E., Crook, JM., Wallace, GG. (2019). Smart graphene-cellulose paper for 2D or 3D "origami-inspired" human stem cell support and differentiation. Colloids and Surfaces B: Biointerfaces 176 (2019) 87-95, https://doi.org/10.1016/j.colsurfb.2018.12.040 Kint, J.; Maier, H.J.; Jagt, E. (2015) Quantification of Infectious Bronchitis Coronavirus by Titration In Vitro and In Ovo. Helena Jane Maier et al. (eds.), Coronaviruses: Methods and Protocols, Methods in Molecular biology, 1282: 89-98. https://dx.doi.org/10.1007%2F978-1-4939-2438-7_9Zhou Chen, R., He, T., Xu, K., Du, D., Zhao, N., Cheng, X., Yang, J., Shi, H., Lin, Y. (2016). Biomedical Potential of Ultrafine Ag/AgCl Nanoparticles Coated on Graphene with Special Reference to Antimicrobial Performances and Burn Wound Healing. ACS Appl. Mater. Interfaces, 8, 15067-15075.https://doi.org/10.1021/acsami.6b03021

## Claims

1. **A HEAT AND MOISTURE EXCHANGE FILTER WITH BIOCIDAL ACTIVITY,** comprising polymeric cellulose fibers impregnated with silver nanoparticles (AgNPs), **characterized in that** the silver nanoparticles comprise a mixture of silver nanoparticles obtained from tannic acid and silver nanoparticles obtained from sodium citrate, wherein the silver nanoparticles obtained from tannic acid have an average size obtained by transmission electron microscopy (TEM) ranging between 10 and 30 nm; and the silver nanoparticles obtained from sodium citrate have an average size obtained by transmission electron microscopy (TEM) ranging between 30 and 50 nm.

2. **A HEAT AND MOISTURE EXCHANGE FILTER WITH BIOCIDAL ACTIVITY** according to claim 1, wherein the polymeric cellulose fiber comprises a heat storage carrier material chosen from calcium chloride, sodium hydroxide and magnesium chloride.

3. **A HEAT AND MOISTURE EXCHANGE FILTER WITH BIOCIDAL ACTIVITY** according to claim 1 or 2, wherein the polymeric cellulose fibers are in the form of a partitioned double layer.

4. **A HEAT AND MOISTURE EXCHANGE FILTER WITH BIOCIDAL ACTIVITY** according to any of claims 1-3, wherein the proportion between the silver nanoparticles obtained from tannic acid and the silver nanoparticles obtained from sodium citrate varies from 1:4 to 4:1.

5. **A HEAT AND MOISTURE EXCHANGE FILTER WITH BIOCIDAL ACTIVITY** according to any of claims 1-4, wherein the silver nanoparticles have an average size obtained by transmission electron microscopy (TEM) ranging between 10 and 55 nm.

6. **A HEAT AND MOISTURE EXCHANGE FILTER WITH BIOCIDAL ACTIVITY** according to any of claims 1-5, wherein the silver nanoparticles have a size obtained by dynamic light scattering (DLS) ranging between 5 and 90 nm.

7. **A HEAT AND MOISTURE EXCHANGE FILTER WITH BIOCIDAL ACTIVITY** according to any of claims 1-6, wherein the silver nanoparticles further comprise a stabilizing agent chosen from polyvinylpyrrolidone (PVP), polyvinyl alcohol, xanthan gum, arabic gum, guar gum, starch and its derivatives, cellulosic derivatives, starches, agar or mixtures thereof.

8. **A HEAT AND MOISTURE EXCHANGE FILTER WITH BIOCIDAL ACTIVITY** according to claim 7, wherein the silver nanoparticles comprise polyvinylpyrrolidone (PVP) stabilizing agent.

9. **A HEAT AND MOISTURE EXCHANGE FILTER WITH BIOCIDAL ACTIVITY** according to any of claims 1-8, wherein the silver nanoparticles are on the surface or immersed in the polymeric cellulose fibers.

10. **A HEAT AND MOISTURE EXCHANGE FILTER WITH BIOCIDAL ACTIVITY** according to any of claims 1-9, wherein the surface of the heat and moisture exchange filter is highly corrugated.

11. **A METHOD FOR PREPARING THE HEAT AND MOISTURE EXCHANGE FILTER WITH BIOCIDAL ACTIVITY** as defined in any of claims 1 to 10, the method comprising the following steps:
a) preparation of silver nanoparticles with tannic acid;
b) preparation of silver nanoparticles with sodium citrate;
c) mixing the nanoparticles obtained in the steps (a) and (b) in a proportion ranging between 1:4 and 4:1;
d) impregnation of cellulose polymeric fibers with the mixture of silver nanoparticles obtained in step (c); and
e) drying the impregnated cellulose fiber obtained in step (d).

12. **A METHOD FOR PREPARING A HEAT AND MOISTURE EXCHANGE FILTER WITH BIOCIDAL ACTIVITY** according to claim 11, wherein step (d) impregnating the cellulose polymeric fibers with the mixture of silver nanoparticles occurs by spraying or immersion.

13. **USE OF SILVER NANOPARTICLES** with biocidal activity in the heat and moisture exchange filter defined in any of claims 1 to 10, wherein the polymeric cellulose fibers that form the heat and moisture exchange filter are impregnated with silver nanoparticles with biocidal activity.

## Patentansprüche

1. **WÄRME- UND FEUCHTIGKEITSAUSTAUSCHFILTER MIT BIOZIDER AKTIVITÄT,** umfassend polymere Cellulosefasern, die mit Silbernanoteilchen (AgNPs) imprägniert sind, **dadurch gekennzeichnet, dass** die Silbernanoteilchen eine Mischung aus aus Gerbsäure erhaltenen Silbernanoteilchen und aus Natriumcitrat erhaltenen Silbernanoteilchen umfassen, wobei die aus Gerbsäure erhaltenen Silbernanoteilchen eine durch Transmissionselektronenmikroskopie (TEM) erhaltene durchschnittliche Größe in einem Bereich zwischen 10 und 30 nm aufweisen; und die aus Natriumcitrat erhaltenen Silbernanoteilchen eine durch Transmissionselektronenmikroskopie (TEM) erhaltene durchschnittliche Größe in dem Bereich zwischen 30 und 50 nm aufweisen.

2. **WÄRME- UND FEUCHTIGKEITSAUSTAUSCHFILTER MIT BIOZIDER AKTIVITÄT** nach Anspruch 1, wobei die polymere Cellulosefaser ein Wärmespeicherträgermaterial umfasst, das aus Calciumchlorid, Natriumhydroxid und Magnesiumchlorid ausgewählt ist.

3. **WÄRME- UND FEUCHTIGKEITSAUSTAUSCHFILTER MIT BIOZIDER AKTIVITÄT** nach Anspruch 1 oder 2, wobei die polymeren Cellulosefasern in der Form einer unterteilten Doppelschicht vorliegen.

4. **WÄRME- UND FEUCHTIGKEITSAUSTAUSCHFILTER MIT BIOZIDER AKTIVITÄT** nach einem der Ansprüche 1 bis 3, wobei das Verhältnis zwischen den aus Gerbsäure erhaltenen Silbernanoteilchen und den aus Natriumcitrat erhaltenen Silbernanoteilchen von 1 : 4 bis 4 : 1 variiert.

5. **WÄRME- UND FEUCHTIGKEITSAUSTAUSCHFILTER MIT BIOZIDER AKTIVITÄT** nach einem der Ansprüche 1 bis 4, wobei die Silbernanoteilchen eine durch Transmissionselektronenmikroskopie (TEM) erhaltene durchschnittliche Größe in dem Bereich zwischen 10 und 55 nm aufweisen.

6. **WÄRME- UND FEUCHTIGKEITSAUSTAUSCHFILTER MIT BIOZIDER AKTIVITÄT** nach einem der Ansprüche 1 bis 5, wobei die Silbernanoteilchen eine durch eine dynamische Lichtstreuung (DLS) erhaltene Größe in dem Bereich zwischen 5 und 90 nm aufweisen.

7. **WÄRME- UND FEUCHTIGKEITSAUSTAUSCHFILTER MIT BIOZIDER AKTIVITÄT** nach einem der Ansprüche 1 bis 6, wobei die Silbernanoteilchen ferner ein Stabilisierungsmittel umfassen, das aus Polyvinylpyrrolidon (PVP), Polyvinylalkohol, Xanthangummi, Gummiarabikum, Guargummi, Stärke und deren Derivaten, Cellulosederivaten, Stärken, Agar oder Mischungen davon ausgewählt ist.

8. **WÄRME- UND FEUCHTIGKEITSAUSTAUSCHFILTER MIT BIOZIDER AKTIVITÄT** nach Anspruch 7, wobei die Silbernanoteilchen ein Polyvinylpyrrolidonstabilisierungsmittel (PVP-Stabilisierungsmittel)umfassen.

9. **WÄRME- UND FEUCHTIGKEITSAUSTAUSCHFILTER MIT BIOZIDER AKTIVITÄT** nach einem der Ansprüche 1 bis 8, wobei die Silbernanoteilchen auf der Oberfläche oder in die polymeren Cellulosefasern eingetaucht sind.

10. **WÄRME- UND FEUCHTIGKEITSAUSTAUSCHFILTER MIT BIOZIDER AKTIVITÄT** nach einem der Ansprüche 1 bis 9, wobei die Oberfläche des Wärme- und Feuchtigkeitsaustauschfilters stark gewellt ist.

11. **VERFAHREN ZUM HERSTELLEN DES WÄRME- UND FEUCHTIGKEITSAUSTAUSCHFILTERS MIT BIOZIDER AKTIVITÄT** nach einem der Ansprüche 1 bis 10, das Verfahren umfassend die folgenden Schritte:
a) Herstellung von Silbernanoteilchen mit Gerbsäure;
b) Herstellung von Silbernanoteilchen mit Natriumcitrat;
c) Mischen der in den Schritten (a) und (b) erhaltenen Nanoteilchen in einem Verhältnis in dem Bereich zwischen 1 : 4 und 4 : 1;
d) Imprägnierung von Cellulosepolymerfasern mit der in Schritt (c) erhaltenen Mischung aus Silbernanoteilchen; und
e) Trocknen der in Schritt (d) erhaltenen imprägnierten Cellulosefaser.

12. **VERFAHREN ZUM HERSTELLEN EINES WÄRME- UND FEUCHTIGKEITSAUSTAUSCHFILTERS MIT BIOZIDER AKTIVITÄT** nach Anspruch 11, wobei Schritt (d) des Imprägnierens der Cellulosepolymerfasern mit der Mischung aus Silbernanoteilchen durch ein Sprühen oder ein Eintauchen erfolgt.

13. **VERWENDUNG VON SILBERNANOTEILCHEN** mit biozider Aktivität in dem Wärme- und Feuchtigkeitsaustauschfilter nach einem der Ansprüche 1 bis 10, wobei die polymeren Cellulosefasern, die den Wärme- und Feuchtigkeitsaustauschfilter ausbilden, mit Silbernanoteilchen mit biozider Aktivität imprägniert sind.

## Revendications

1. **FILTRE D'ÉCHANGE DE CHALEUR ET D'HUMIDITÉ À ACTIVITÉ BIOCIDE,** comprenant des fibres de cellulose polymères imprégnées de nanoparticules d'argent (AgNP), **caractérisé en ce que** les nanoparticules d'argent comprennent un mélange de nanoparticules d'argent obtenues à partir d'acide tannique et de nanoparticules d'argent obtenu à partir de citrate de sodium, dans lequel les nanoparticules d'argent obtenues à partir d'acide tannique ont une taille moyenne obtenue par microscopie électronique à transmission (MET) comprise entre 10 et 30 nm ; et les nanoparticules d'argent obtenues à partir du citrate de sodium ont une taille moyenne obtenue par microscopie électronique à transmission (MET) comprise entre 30 et 50 nm.

2. **FILTRE D'ÉCHANGE DE CHALEUR ET D'HUMIDITÉ À ACTIVITÉ BIOCIDE** selon la revendication 1, dans lequel la fibre de cellulose polymère comprend un matériau porteur de stockage de chaleur choisi parmi chlorure de calcium, hydroxyde de sodium et chlorure de magnésium.

3. **FILTRE D'ÉCHANGE DE CHALEUR ET D'HUMIDITÉ À ACTIVITÉ BIOCIDE** selon la revendication 1 ou 2, dans lequel les fibres de cellulose polymères sont sous la forme d'une double couche cloisonnée.

4. **FILTRE D'ÉCHANGE DE CHALEUR ET D'HUMIDITÉ À ACTIVITÉ BIOCIDE** selon l'une quelconque des revendications 1 à 3, dans lequel la proportion entre les nanoparticules d'argent obtenues à partir de l'acide tannique et les nanoparticules d'argent obtenues à partir du citrate de sodium varie de 1:4 à 4:1.

5. **FILTRE D'ÉCHANGE DE CHALEUR ET D'HUMIDITÉ À ACTIVITÉ BIOCIDE** selon l'une quelconque des revendications 1 à 4, dans lequel les nanoparticules d'argent ont une taille moyenne obtenue par microscopie électronique à transmission (MET) comprise entre 10 et 55 nm.

6. **FILTRE D'ÉCHANGE DE CHALEUR ET D'HUMIDITÉ À ACTIVITÉ BIOCIDE** selon l'une quelconque des revendications 1 à 5, dans lequel les nanoparticules d'argent ont une taille obtenue par diffusion dynamique de lumière (DLS) comprise entre 5 et 90 nm.

7. **FILTRE D'ÉCHANGE DE CHALEUR ET D'HUMIDITÉ À ACTIVITÉ BIOCIDE** selon l'une quelconque des revendications 1 à 6, dans lequel les nanoparticules d'argent comprennent en outre un agent stabilisant choisi parmi la polyvinylpyrrolidone (PVP), l'alcool polyvinylique, la gomme de xanthane, la gomme arabique, la gomme de guar, l'amidon et ses dérivés, dérivés cellulosiques, amidons, gélose ou mélanges de ceux-ci.

8. **FILTRE D'ÉCHANGE DE CHALEUR ET D'HUMIDITÉ À ACTIVITÉ BIOCIDE** selon la revendication 7, dans lequel les nanoparticules d'argent comprennent un agent stabilisant de polyvinylpyrrolidone (PVP).

9. **FILTRE D'ÉCHANGE DE CHALEUR ET D'HUMIDITÉ À ACTIVITÉ BIOCIDE** selon l'une quelconque des revendications 1 à 8, dans lequel les nanoparticules d'argent sont à la surface ou immergées dans les fibres de cellulose polymères.

10. **FILTRE D'ÉCHANGE DE CHALEUR ET D'HUMIDITÉ À ACTIVITÉ BIOCIDE** selon l'une quelconque des revendications 1 à 9, dans lequel la surface du filtre d'échange de chaleur et d'humidité est hautement ondulée.

11. **PROCÉDÉ DE PRÉPARATION DU FILTRE D'ÉCHANGE DE CHALEUR ET D'HUMIDITÉ AVEC UNE ACTIVITÉ BIOCIDE** selon l'une quelconque des revendications 1 à 10, le procédé comprenant les étapes suivantes :
a) la préparation de nanoparticules d'argent avec de l'acide tannique ;
b) la préparation de nanoparticules d'argent avec du citrate de sodium ;
c) le mélange des nanoparticules obtenues aux étapes (a) et (b) dans une proportion comprise entre 1:4 et 4:1 ;
d) l'imprégnation des fibres polymères de cellulose avec le mélange de nanoparticules d'argent obtenu à l'étape (c) ; et
e) le séchage de la fibre de cellulose imprégnée obtenue à l'étape (d).

12. **PROCÉDÉ DE PRÉPARATION D'UN FILTRE D'ÉCHANGE DE CHALEUR ET D'HUMIDITÉ AVEC UNE ACTIVITÉ BIOCIDE** selon la revendication 11, dans lequel l'étape (d) d'imprégnation des fibres polymères de cellulose avec le mélange de nanoparticules d'argent se produit par pulvérisation ou immersion.

13. **UTILISATION DE NANOPARTICULES D'ARGENT** ayant une activité biocide dans le filtre d'échange de chaleur et d'humidité selon l'une quelconque des revendications 1 à 10, dans laquelle les fibres de cellulose polymères qui forment le filtre d'échange de chaleur et d'humidité sont imprégnées de nanoparticules d'argent ayant une activité biocide.
